# EUROPEAN PATENT APPLICATION

(11) **EP 1 618 884 A1**
(43) Date of publication of application: **25.01.2006**
(21) Application number: 04729505.0
(22) Date of filing: 26.04.2004
(51) Int. Cl.: A61K 35/74, A61K 47/32, A61K 47/34, A61K 47/38, A61K 47/48, A61P 19/02, A61P 29/00, A61P 37/02, A61P 37/08, A61P 35/00, A61P 37/04, A61P 43/00

(54) **INSTRUMENT FOR INDUCING CYTOKINE AND METHOD OF INDUCING CYTOKINE**

(30) Priority: 28.04.2003 JP 2003124344; 18.07.2003 JP 2003276831
(71) Applicant: SEKISUI CHEMICAL CO., LTD., Osaka-shi, Osaka 530-8565 (JP)
(72) Inventor: KITAHARA, S., Sekisui Chemical Co., Ltd, Mishima-gun,Osaka 6188589 (JP); SHINMURA, K., Sskisui Chemical Co., Ltd., Mishima-gun,Osaka 6188589 (JP); ABE, Y., Sekisui Chemical Co., Ltd., Mishima-gun Osaka 6188589 (JP); KURIYAMA, K., Sekisui Chemical Co., Ltd., Mishima-gun Osaka 6188589 (JP)
(74) Representative: Merkle, Gebhard
(86) International application number: PCT/JP2004/005983
(87) International publication number: WO 2004/096247

(57) **Abstract**

It is intended to provide a novel instrument for inducing a cytokine and a method of inducing a cytokine by which a cytokine can be effectively induced compared to the existing cytokine induction therapy. Namely, an instrument for inducing a cytokine which contains a hemolytic streptococcus and/or a hemolytic streptococcus-origin component and a water-insoluble carrier; and a method of inducing a cytokine by using this instrument for inducing a cytokine.

## Description

### TECHNICAL FIELD

The present invention relates to an instrument for inducing cytokine and a method for inducing cytokine, which are used in a cytokine-inducing therapy or the like and are enable effective induction of cytokines.

### BACKGROUND ART

Cytokine is a general term for a diversity of factors of intercellular signal transductions. Examples of cytokines include interferon-α, interferon-β, interferon-γ (IFN-γ) , interleukin 1 to interleukin 27, tumor necrosis factor-α (TNF-α) , tumor necrosis factor-β, transforming growth factor-α, transforming growth factor-β (TGF-β), various cell growth factors and the like (Special 1995 number of Clinical Immunity Vol. 27, Suppl. 16, "All of cytokines", Kagaku Hyoron-sha, Clinical Immunity Vol.36, 39-44, 2001, and Clinical Immunity Vol. 39, 189-200, 2003).

Cytokine is known to exhibit various activities in vivo and be involved in various diseases. A cytokine-inducing therapy has been conventionally conducted which causes such activities of cytokine in vivo to thereby treat for diseases. In the cytokine-inducing therapy, a cytokine inducer is dosed to a patient to cause induction of cytokine in vivo. Various substances are known to serve as a substance inducing cytokine for use in such a cytokine-inducing therapy. Examples of known substances inducing cytokine include microorganism-derived substances such as OK-432, Mycobacteria bovis bacillus Calmette-Guerin (BCG), Bestatin, SSM (Supecific Substance Maruyama) and Romurtide, and basidiomycetes-derived substances such as Krestin, Lentinan and Sizofiran.

For example, OK-432, BCG and the like are known to induce cytokines such as interleukin 1 and interferon-γ, from blood etc. (Gifu University Medical Report 43: 166-177, 1995, Molecular Medicine Vol.36, extra edition, 220-229, 1999).

Although possible to induce cytokines in vivo, the above-described cytokine-inducing therapy is hard to induce cytokines in a sufficient amount and is accordingly difficult to provide their strong efficacies, which has been a problem. A dosage of a cytokine inducer may be increased to effectively induce cytokines. However, the increased dosage heightens side effects to result in the failure to achieve effective therapy.

Meanwhile, Japanese Patent Application Laid-Open No. 61-277628 describes a leukocyte stimulator for the treatment of cancer, which comprises OK-432, which is one of hemolytic streptococcus covalently bonded to an insoluble carrier. This leukocyte stimulator induces tumor cytotoxic cells. This prior art reference provides no description as to a cytokine-inducing instrument and a method for inducing cytokine.

### DISCLOSURE OF THE INVENTION

In view of the current state of the above-described prior art, it is an object of the present invention to provide a novel cytokine-inducing instrument and a method for inducing cytokine, which enable more effective induction of cytokine relative to conventional cytokine-inducing therapies.

The present invention has been done to accomplish the above-mentioned object. The instrument for inducing a cytokine of the present invention is characterized in that said instrument comprises hemolytic streptococcus and/or a hemolytic streptococcus-origin component and a water-insoluble carrier.

Furthermore, the method for inducing a cytokine of the present invention is characterized in that said method comprises inducing a cytokine using the instrument for inducing a cytokine constituted according to the present invention.

The present inventors have found that hemolytic streptococcus and/or a hemolytic streptococcus-origin component and a water-insoluble carrier show remarkably high amount of induction of a cytokine, which resulted in the completion of the present invention.

Hereinafter, the present invention is explained in detail.

The instrument for inducing a cytokine of the present invention comprises hemolytic streptococcus and/or a hemolytic streptococcus-origin component and a water-insoluble carrier.

The carrier may be any water-insoluble carrier and is not specifically limited. Examples of the material which constitutes the carrier include inorganic materials, organic materials, metals and the like, preferably inorganic materials and organic materials. Among the inorganic materials, carbon materials are preferred, and among the organic materials, polymer materials are preferred. Among these, active carbon is further preferred.

Examples of the inorganic materials include carbon materials such as active carbon, glass or glass derivatives, silica-based compositions, alumina and hydroxyapatite. Among these, carbon materials are preferable, and among the carbon materials, active carbon is preferable.

The carbon material is a carbonized substance obtained by calcining an organic compound according to any conventionally known method. The active carbon is obtained by forming pores on the carbonized substance via activation. The method for activation is not specifically limited, and any conventionally known method can be used. Examples of such method include a method comprising activating by vapor at high temperature, a method comprising activating using a chemical substance, and the like. It is preferable that the activation is carried out so as to adjust the specific surface area to not less than 800 m²/g.

The organic compounds used as a raw material for the carbon material are not specifically limited. Among these, examples of the raw material for active carbon include petroleum pitch, coal, wood, coconut husk, sawdust, and synthetic resins such as phenolic resins, vinyl chloride resins, acrylic resins and vinylidene chloride resins. Among these, petroleum pitch, coal, coconut husk and phenolic resins are preferable, and petroleum pitch and phenolic resins are more preferable.

The active carbon is in various forms such as spherical or non-spherical, and the form is not specifically limited. Examples of the non-spherical form include a pellet form, a columnar form, a crushed form, a fibrous form and the like. The active carbon may be optionally coated with a water-insoluble polymer material such as polyhydroxyethyl methacrylate, nitro cellulose or hydroxyethylcellulose.

The diameter of the active carbon is preferably more than 100 µm and not more than 10000 µm. Where the active carbon is in spherical form, the diameter of active carbon means particle diameter. Alternatively, where the active carbon is in non-spherical form other than spherical form, the diameter means that at least one of the major axis diameter and minor axis diameter is preferably more than 100 µm and not more than 10000 µm. More preferably, where the active carbon is in non-spherical form, both the major axis diameter and minor axis diameter are more than 100 µm and not more than 10000 µm.

Where the active carbon is in spherical form, it is preferable that the lower limit of the particle diameter is more than 100 µm, because the effect of enhancing induction of a cytokine is decreased where the particle diameter is not more than 100 µm; and the upper limit is preferably 10000 µm, more preferably 2000 µm. The spherical does not necessarily mean a perfect sphere. Where the active carbon is in incomplete spherical form, a sphere in which the active carbon is housed while contacting therein is hypothesized, and the particle diameter of said active carbon is calculated from the diameter of said hypothetical sphere.

Where the active carbon is in non-spherical form other than fibrous form, it is preferable that the lower limit of the major axis diameter is more than 100 µm, because the effect of enhancing induction of a cytokine is decreased where the major axis diameter is not more than 100 µm; and the upper limit is preferably 10000 µm, more preferably 4000 µm. The lower limit of the minor axis diameter is preferably more than 100 µm; and the upper limit of minor axis diameter is 10000 µm, more preferably 2000 µm. The major axis diameter and minor axis diameter of the active carbon in a non-spherical form is obtained by hypothesizing a column in which the active carbon is housed while contacting therein, wherein the height direction of the column corresponds to the most widest width of the active carbon having a non-spherical shape, and calculating the major axis diameter from the height of the column and the minor axis diameter from the diameter of the circle on the bottom of the column. The major axis diameter and minor axis diameter may be the same value.

The particle diameter, major axis diameter and minor axis diameter as referred in the present invention may be actually measured after magnifying the active carbon using an electromicroscope or the like. Alternatively, the active carbon having the particle diameter, major axis diameter and minor axis diameter within the range as defined by the present invention can be obtained by the following screening method.

Firstly, stainless screens having an inner diameter of 200 mm each having the maximum diameter of screen opening of 100 µm, 2000 µm, 4000 µm and 10000 µm are prepared. Meanwhile, a sample in the amount of 10 to 100 g is collected from an active carbon whose diameter is to be measured. Where the active carbon is aggregated due to moisture absorption by the sample, the sample is dried under such condition that the original characteristic of the sample is not deteriorated.

Secondly, the screen having the maximum diameter of screen opening of 2000 µm is stacked on the screen having the maximum diameter of screen opening of 100 µm; the screen having the maximum diameter of screen opening of 4000 µm is stacked thereon; and the screen having the maximum diameter of screen opening of 10000 µm is further stacked thereon. Thereafter, the sample is put in the screen having the maximum diameter of screen opening of 10000 µm on the top, and screening is carried out for 5 minutes using the stack of screens all at once.

After the screening is completed, the residue on the screen is collected as follows. Where the active carbon is in spherical form, the residue on the screen having the maximum diameter of screen opening of 100 µm is an active carbon having a particle diameter from more than the lower limit of 100 µm to the upper limit of 2000 µm, and the combined residue on the screens each having the maximum diameter of screen opening of 100 µm, 2000 µm and 4000 µm is an active carbon having a particle diameter from more than the lower limit of 100 µm to the upper limit of 10000 µm. Where the active carbon is in non-spherical form, the residue on the screen having the maximum diameter of screen opening of 100 µm is an active carbon having a major axis diameter from more than the lower limit of 100 µm to the upper limit of 4000 µm and a minor axis diameter from more than the lower limit of 100 µm to the upper limit of 2000 µm; and the combined residue on the screens each having the maximum diameter of screen opening of 100 µm, 2000 µm and 4000 µm is an active carbon having a major axis diameter from more than the lower limit of 100 µm to the upper limit of 10000 µm and a minor axis diameter from more than the lower limit of 100 µm to the upper limit of 10000 µm. During the screening method, an active carbon having a diameter other than the particle diameter, the major axis diameter and the minor axis diameter may be incorporated, but it is permissible so long as the amount of said active carbon is substantially negligible.

The active carbon may be in a fibrous form. In such case, an active carbon in known form such as fabric form, net form, sheet form or hollow fiber form can be used.

Examples of the polymer materials include materials such as cellulose-based materials, agarose-based materials, dextran-based materials, polystylene-based materials, polyester-based materials (e.g., polyacrylic ester-based materials and polyethylene terephthalate-based materials), polyamide-based materials (e.g., nylon-based materials), polyvinyl alcohol-based materials, polysulfone-based materials, polyacrylonitrile-based materials, polyurethane-based materials, polyethylene-based materials, polypropylene-based materials and polyvinyl chloride-based materials. Among these, cellulose-based materials, polystyrene-based materials, polyacrylic ester-based materials, polyester-based materials, nylon-based materials, polyvinyl alcohol-based materials, polypropylene-based materials and polyvinyl chloride-based materials are preferable, and polystyrene-based materials, polyacrylic ester-based materials, polypropylene-based materials and polyvinyl chloride-based materials are more preferable.

Examples of the polystyrene-based materials include divinylbenzene-styrene copolymer and the like, and examples of the polyacrylic ester-based polymer material include polymethyl methacrylate and polyhydroxyethyl methacrylate.

Where the carrier comprises a polymer material and is in the form of particle, the particle diameter is preferably from the lower limit of 100 µm to the upper limit of 20000 µm. Where the polymer material is in fibrous form, the fiber diameter is preferably not more than 10 µm, more preferably not more than 5 µm. Where the carrier comprises a fibrous polymer material, it is preferably non-woven fabric, and the fiber diameter of the non-woven fabric is preferably not more than 3 µm.

Examples of the metals include gold or gold alloy, silver or silver alloy, titanium or titanium alloy, stainless and the like.

For a leukocyte stimulator for treatment of cancer disclosed in JP-A NO.61-277628, it is considered to be preferable to use a carrier on which leukocyte cannot be adsorbed, whereas the carrier used for the present invention preferably comprises a material on which leukocyte and the like can be adsorbed. Examples of the material which absorbs leukocyte include inorganic materials such as carbon materials (e.g., active carbon, etc.) and glass or glass derivatives, and organic materials such as polymer materials such as polystyrene-based materials, polyacrylic ester-based materials, polyester-based materials, nylon-based materials, polyvinyl alcohol-based materials, polypropylene-based materials, polyvinyl chloride-based materials and cellulose-based materials (e.g., cellulose acetate, etc.). Among these, active carbon and polymer materials such as polystyrene-based materials, polyacrylic ester-based materials, polypropylene-based materials and polyvinyl chloride-based materials are preferable, and active carbon is more preferable.

The carrier is nonpolar, and may be hydrophobic or hydrophilic. Where the carrier is hydrophobic, it comprises preferably active carbon, or a polystylene-based, polyacrylic ester-based, polypropylene-based or polyvinyl chloride-based polymer material, more preferably active carbon. Furthermore, hydrophilicity can be provided to the surface of the carrier by means of surface modification, surface coating or the like.

The form of the carrier is not specifically limited, and a known form such as a particle form, fibrous form, non-woven fabric form, sponge form, membrane form, sheet form or hollow fiber form or the like can be used. Among these, a particle form and a fibrous form are preferable.

The carrier preferably has a surface roughness, and the surface roughness is preferably due to porosity and the like of the constitutional material. The porous constitutional material used for the carrier is preferably active carbon, as well as polymer materials such as polystyrene-based materials, polyacrylic ester-based materials, polyester-based materials, nylon-based materials, cellulose-based materials, polyvinyl alcohol-based materials, polypropylene-based materials and polyvinyl chloride-based materials, preferably active carbon.

The surface roughness of the carrier may be due to fibrous shape where the constitutional material is fibrous material, and in such case, the carrier is preferably in the form of non-woven fabric comprising fibrous material. Examples of the fibrous material include carbon materials such as active carbon, as well as polymer materials such as polystyrene-based materials, polyacrylic ester-based materials, polyester-based materials, nylon-based materials, cellulose-based materials, polyvinyl alcohol-based materials, polypropylene-based materials and polyvinyl chloride-based materials, preferably active carbon and polymer materials such as polystyrene-based materials, polyacrylic ester-based materials, polypropylene-based materials and polyvinyl chloride-based materials. A carrier comprising at least one kind of these preferable polymer materials is preferably used, and the material which constitutes the carrier is more preferably active carbon.

The hemolytic streptococcus is bacterium which belongs to hemolytic streptococcus, preferably pyogenetic streptococcus which shows Group A Type β hemolytic property, more preferably bacterium which belongs to *Streptococcus Pyogenes.* For example, OK-432, which is a fungal form comprising lyophilized powder of *Streptococcus Pyogenes* (Group A Type β) Su strain, treated with penicillin (JP-A NO. 49-48822).

Examples of the hemolytic streptococcus-origin component include OK-PSA (J. Immunother. 2000, Jan; 23 (1) , 94-103) , which is an OK-432-origin component obtained by extraction and purification by affinity chromatography using particles in which a monoclonal antibody has been bonded to a CNBr-activated cephallose as a column carrier, a soluble component and an insoluble component to an organic solvent of hemolytic streptococcus, which are obtained by contacting hemolytic streptococcus with an organic solvent such as methanol, ethanol, acetone, ethyl acetate, diethylether, dichloromethane, chloroform or the like. Furthermore, a soluble component and an insoluble component to an aqueous solution of hemolytic streptococcus, which is obtained by contacting hemolytic streptococcus with water, physiological saline or an aqueous solution of an inorganic salt of phosphorous, potassium, magnesium, calcium or the like, and the like are also included in the hemolytic streptococcus-origin component for the present invention.

Where the hemolytic streptococcus and/or the hemolytic streptococcus-origin component alone cannot exhibit sufficient ability of inducing a cytokine, an activity of inducing a cytokine can be exhibited by using it as a combination with the water-insoluble carrier.

It is preferable that the hemolytic streptococcus and/or the hemolytic streptococcus-origin component have been fixed on the surface of the carrier. Fixing can be carried out by a conventional method such as physical adsorption, covalent bonding or ionic bonding. Physical adsorption is preferable in view of easiness of fixing. Where covalent bonding is used, a spacer having a given length can be optionally introduced in the bonding portion between the hemolytic streptococcus and/or the hemolytic streptococcus-origin component and the carrier.

The hemolytic streptococcus and/or the hemolytic streptococcus-origin component may be optionally subjected to various pre-treatment such as washing operation, crushing operation or fractionation operation of components of bacterium form prior to fixing. Although OK-432 is living bacterium which does not have ability of proliferation, in order to increase safety, OK-432 may be optionally killed by various method such as heat treatment, chemical treatment, radiation treatment or gas sterilization treatment at any period before or after fixing or at the same time as fixing. Examples of the heating method include autoclave treatment and the like; examples of the chemical treatment include treatments using glutalaldehyde treatment, formalin treatment, ethanol treatment and the like; examples of the radiation treatment include γ-ray treatment and the like; and examples of the gas sterilization treatment include ethylene oxide gas treatment and the like. Among these treatments, formalin treatment, which is chemical treatment, is preferable, because said treatment has been conventionally used as an antiseptic solution or the like, is safe, and can remarkably stabilize the bacterium form itself.

The hemolytic streptococcus and/or the hemolytic streptococcus-origin component may be fixed on the carrier, by binding the component through the amino acid, sugar components or the like in the outer wall component on the surface of the bacterium form to functional groups such as carboxylic group, amino group and/or epoxy group on the carrier. During this procedure, spacers having various chain length and structure can be optionally introduced.

Where the surface of the hemolytic streptococcus and/or the hemolytic streptococcus-origin component is electrically charged by the treatment, it can be fixed on a carrier having counter electrical charge on its surface by ionic bonding action.

In the present invention, the instrument for inducing a cytokine preferably further comprises a container which comprises hemolytic streptococcus and/or the hemolytic streptococcus-origin component. The present invention also encompass a method for inducing a cytokine, which comprises inducing a cytokine by adding a cell which produces cytokine to a container which constitutes the instrument for inducing a cytokine of the present invention.

Where the hemolytic streptococcus and/or the hemolytic streptococcus-origin component and the carriers are contained in the container, these are preferably contained together in the container. Namely, a constitution in which the cell which produces cytokine, hemolytic streptococcus and/or a hemolytic streptococcus-origin component and carriers are simultaneously contacted each other is preferable. However, for the present invention, the container is not always required, and an instrument having no container may be used. Furthermore, where a container is used, the container is not necessarily constituted so that the hemolytic streptococcus and/or the hemolytic streptococcus-origin component and the carrier can be contacted with the cell which produces a cytokine in said container.

The cell which produces a cytokine widely encompasses various cells by which production of cytokines is induced using the instrument for inducing a cytokine of the present invention. Examples of such cell which produces a cytokine include blood, blood constituents and various cells which can produce cytokines, such as leucocyte and blood platelet which are separated from blood, as well as cells collected from tissues such as bone marrow cells, dendritic cells, epidermic cells, fibroblast cells, hepatic cells, osteoblast, blood stem cells, embryonic stem cells or the like, cultured cells, established cell lines, various cells which produce cytokines, or the like.

The blood used for the instrument for inducing a cytokine of the present invention of the present invention and the method for inducing a cytokine refers to blood and diluted blood obtained by diluting blood with a suitable dilution solution such as physiological saline, medium or buffer. An anticoagulating agent and additives may be added to the blood.

The blood constituent used for the instrument for inducing a cytokine and the method for inducing a cytokine of the present invention refers to leucocyte and blood platelet which are separated from blood, as well as constitutions those comprising bone marrow cells, dendritic cells, blood stem cells, established cell lines derived from blood cell, or the like. These can be used solely or as a mixture with leucocyte and the like which are separately collected and blood and the like. Alternatively, these constitutions may be diluted with a suitable diluting liquid such as physiological saline, medium or buffer and used for the present invention. Preferably, blood is used.

In the following explanation, for the purpose of plain explanation, examples using blood or a blood constituent or the like are used as a cell which produces cytokine.

Although the ratio of the carrier used is not specifically limited, where a carrier in the form of particle is used, the bulk volume amount of the carrier relative to the volume of blood or the blood constituent or the like is preferably 0.02% at the lower limit, more preferably 0.1% at the lower limit, and preferably 80% at the upper limit, more preferably 50% at the upper limit.

Although the ratio of the hemolytic streptococcus and/or the hemolytic streptococcus-origin component to be used is not specifically limited, for example, where OK-432 is used, the concentration to be added of blood or the blood constituent or the like is preferably 0.0001 KE/mL at the lower limit and preferably 10 KE/mL at the upper limit.

Where a cytokine is induced using the instrument for inducing a cytokine of the present invention, blood or the blood constituent or the like is contacted in the container comprising the carrier and the hemolytic streptococcus and/or the hemolytic streptococcus-origin component, whereby a cytokine is effectively induced in the blood or blood constituent or the like. In this case, the temperature for the contact is preferably 15°C at the lower limit and 42°C at the upper limit, whereby a cytokine can be induced more effectively.

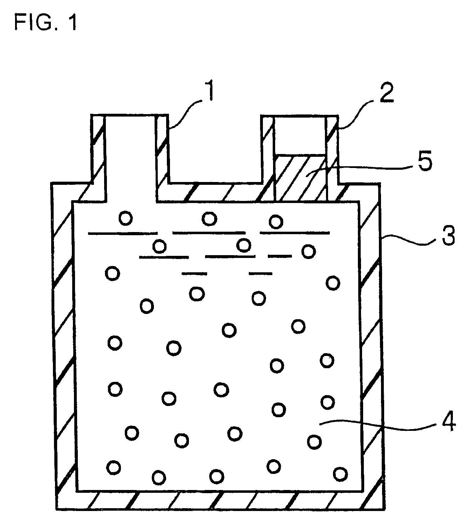

Although the structure of the container preferably used for the instrument for inducing a cytokine of the present invention is not specifically limited, as schematically shown in Fig. 1, a container 3 comprising an introduction portion 1 for blood or the blood constituent or the like and a derivation portion 2 which derives blood or blood constituent or the like 4 where cytokine has been induced out of the container is preferable.

As the container, a hard columnar container, a soft blood bag container and the like are more preferable.

Furthermore, where blood or the blood constituent or the like in which a cytokine has been induced is derived to the outside of the container, the container used for the instrument for inducing a cytokine of the present invention preferably has an effusion-preventing structure so that the water-insoluble carrier and hemolytic streptococcus and/or the hemolytic streptococcus-origin component, which constitute the instrument for inducing a cytokine, are not incorporated in the blood or blood constituent or the like.

As schematically shown in Fig.1, the effusion preventing structure 5 may be fixed in the container in advance so that the water-insoluble carrier and hemolytic streptococcus and/or the hemolytic streptococcus-origin component, which constitute the instrument for inducing a cytokine, are not detached. In this case, examples of the effusion preventing structure include a separation membrane, a separation filter and the like for preventing effusion. Examples of other effusion-preventing structure include a structure for separating from blood or the blood constituent or the like by centrifugation operation or the like.

If desired, a fluid constituent or the like is separated from blood or the blood constituent or the like in which a cytokine has been induced, which may be used for treatment or the like.

In one embodiment of the instrument for inducing a cytokine of the present invention, for example, blood or the blood constituent or the like is introduced into the instrument for inducing a cytokine of the present invention constituted by a blood bag having an introduction portion and a derivation portion and a carrier in the form of particle, fiber or non-woven fabric on which OK-432 has been fixed. Where necessary, the blood or the blood constituent or the like on which a cytokine has been induced can be collected from the derivation portion and used.

The cytokine induced by the method for inducing a cytokine of the present invention using the instrument for inducing a cytokine of the present invention is not specifically limited, and preferable examples thereof include interferon-γ (TFN-γ) , interleukin 10 (IL-10), interleukin 12 (IL-12), tumor necrosis factor-α (TNF-α) and the like. As is apparent from the Examples mentioned below, more preferable cytokine is interferon-γ (IFN-γ) , because the amount of the cytokine induced is increased by the carrier. IFN-γ is a cytokine which plays very important role in various diseases such as allergic diseases and cancer. By inducing IFN-γ, a treatment effect for these diseases can be expected.

The instrument for inducing a cytokine of the present invention is an innovative instrument which can induce a cytokine of an amount in practical level using hemolytic streptococcus and/or the hemolytic streptococcus-origin component in the co-presence of a water-insoluble carrier. The present invention can induce a considerably higher level of amount of a cytokine in a container than that of the case where hemolytic streptococcus and/or the hemolytic streptococcus-origin component is solely used in a container. Therefore, the present invention can be preferably used for the treatment of various diseases to which cytokines are effective. Furthermore, the instrument for inducing a cytokine of the present invention induces a cytokine by contacting with blood or blood constituent or the like outside of the body, which can be used for the treatment after the hemolytic streptococcus and/or the hemolytic streptococcus-origin component or the like which has a possibility of developing side effect after induction of a cytokine is removed, if necessary. Accordingly, a treatment which hardly provides side effects can be accomplished with higher safety as compared to a method comprising administering hemolytic streptococcus and/or a hemolytic streptococcus-origin component.

### BRIEF DESCRIPTION OF DRAWINGS

Fig. 1 is a schematic cross-sectional view which shows an example of the instrument for inducing a cytokine of the present invention.

### BEST MODE FOR CARRYING OUT THE INVENTION

The following examples illustrate the present invention more specifically but are not intended to be limiting to the examples.

IFN-γ in human plasma and rat plasma were quantified by using an ELISA kit manufactured by R&D Systems, an ELISA kit manufactured by Endogen and an ELISA kit manufactured by Genzyme Techne.

The IFN-γ value of plasma in blood from a healthy human immediately after collection of blood and treatment of blood only according to Example 1 mentioned below were both not more than 10 pg/mL. Similarly, the IFN-γ value of plasma in blood from a rat immediately after collection of blood and treatment of blood only according to Example 1 mentioned below were both not more than 40 pg/mL.

### (Example 1)

Carrier 1 (particulate polystyrene-based polymer material, manufactured by Mitsubishi Chemical Corp., trade name: DIAION HP-50) was washed via decantation with methanol (manufactured by Wako Pure Chemicals Industries, Ltd. , for HPLC use) and thereafter decantation with purified water (manufactured by Otsuka Pharmaceutical Co., Ltd.). The carrier 1 was thereafter washed via decantation with physiological saline for injection (manufactured by Otsuka Pharmaceutical Co., Ltd.). The carrier 1 in the particle bulk volume of 20 µL was then packed in a sterilized tube (manufactured by DIATRON Corp., Eppendorf tube for 1.5 ml use).

Blood was collected from a healthy human to prepare venous blood containing 15 IU/ml of heparin. OK-432 (manufactured by Chugai Pharmaceutical Co., Ltd., Trade Name: Picibanil) was added in the concentration of 0.1 KE/mL of blood. OK-432 as used herein was prepared with physiological saline so that the ratio by volume of the physiological saline to blood became 1% . The OK-432-containing blood was added to the tube packed with the carrier 1 in the bulk volume of 20 µL so that a tube scale became 1.5 mL. Namely, the blood was added by the amount of 1.48 mL.

Secondly, the tube was tumbled to stir the blood and then attached to a rotary mixer (manufactured by TAITECH Corp.), which was subsequently rotated at 6 rpm in a constant temperature vessel to effect incubation at 37°C for 24 hours. After incubation, the blood was centrifuged at 3,500 rpm (manufactured by Tomy Seiko Co., Ltd., micro high-speed centrifuge MRX-150) at 4°C for 15 minutes. Blood plasma was then collected from the blood and freezed at -20°C for preservation. The preserved plasma was then melted and the amount of IFN-γ induced in the plasma was determined using a Human IFN-γ ELISA kit (manufactured by R&D Systems or ENDOGEN) . The results are shown in Table 1.

### (Examples 2 and 3)

The amounts of IFN-γ induced were determined according to a similar manner to Example 1, except that the bulk volume of carrier 1 were a predetermined amounts as shown in Table 1 and the amount of the OK-432-containing blood added to the tube was 1.5 mL (based on the bulk volume of carrier 1). The results are shown in Table 1.

### (Comparative Example 1)

The amount of IFN-γ induced was determined according to a similar manner to Example 1, except that carrier 1 was not used and the amount of the OK-432-containing blood added to the tube was 1.5 mL. The results are shown in Table 1.

### (Comparative Examples 2 to 4)

The amounts of IFN-γ induced were determined according to similar manners to Examples 1 to 3, respectively, except that OK-432 was not added to the blood. The results are shown in Table 1.

**Table 1**

| | Carrier 1 | Concentration of OK-432 | Amount of IFN-γ induced |
|---|---|---|---|
| | Bulk volume (µL) | (KE/mL) | (pg/mL) |
| Example 1 | 20 | 0.1 | 360 |
| Example 2 | 50 | 0.1 | 367 |
| Example 3 | 100 | 0.1 | 323 |
| Comparative Example 1 | - | 0.1 | 114 |
| Comparative Example 2 | 20 | - | <10 |
| Comparative Example 3 | 50 | - | <10 |
| Comparative Example 4 | 100 | - | <10 |

### (Example 4)

The amount of IFN-γ induced in the plasma was determined according to a similar manner to Example 2, except that carrier 2 (particulate polystyrene-based polymer material, manufactured by Organo Corporation, trade name: Amberlite XAD-2000) was used instead of carrier 1. The results are shown in Table 2.

### (Example 5)

The amount of IFN-γ induced was determined according to a similar manner to Example 4, except that the bulk volume of carrier 2 was 100 µL and the amount of the OK-432-containing blood added to the tube was 1.4 mL. The results are shown in Table 2.

### (Comparative Example 5)

The amount of IFN-γ induced was determined according to a similar manner to Example 4, except that carrier 2 was not used and the amount of the OK-432-containing blood added to the tube was 1.5 mL. The results are shown in Table 2.

### (Comparative Examples 6 and 7)

The amounts of IFN-γ induced were determined according to similar manners to Examples 4 and 5, respectively, except that OK-432 was not added to the blood. The results are shown in Table 2.

**Table 2**

| | Carrier 2 | Concentration of OK-432 | Amount of IFN- γ induced |
|---|---|---|---|
| | Bulk volume (µL) | (KE/mL) | (pg/mL) |
| Example 4 | 50 | 0.1 | 251 |
| Example 5 | 100 | 0.1 | 236 |
| Comparative Example 5 | - | 0.1 | 117 |
| Comparative Example 6 | 50 | - | <10 |
| Comparative Example 7 | 100 | - | <10 |

Secondly, induction of a cytokine in rat blood was investigated in Examples 6 and 7 and Comparative Examples 8 to 10.

### (Example 6)

A sterilized tube packed with the carrier 1 in the bulk volume of 50 µL was obtained according to a similar manner to Example 2.

Venous blood containing 15 IU/ml of heparin was collected from a Wister rat (7 weeks old, male, purchased from Japan SLC, Inc.). OK-432 (manufactured by Chugai Pharmaceutical Co., Ltd., Trade name: Picibanil) was added to the blood in the concentration of 0.1 KE/mL. This OK-432 was prepared using an RPMI 1640 medium. The ratio of the RPMI 1640 medium to blood was prepared to be 20%. The OK-432-containing blood was added to the tube, packed with the carrier 1 until a tube scale indicated 1.5 mL.

Secondly, the procedure of Example 2 was followed to collect blood plasmas and determine the amount of IFN-γ induced therein using a rat IFN-γ quantification kit (manufactured by Genzyme Techne). The results are shown in Table 3.

### (Example 7)

The amount of IFN-γ induced was determined according to a similar manner to Example 6, except that the bulk volume of carrier 1 was 500 µL and the amount of the OK-432-containing blood added to the tube was 1.0 mL. The results are shown in Table 3.

### (Comparative Example 8)

The amount of IFN-γ induced was determined according to a similar manner to Example 6, except that carrier 1 was not used and the amount of the OK-432-containing blood added to the tube was 1.5 mL. The results are shown in Table 3.

### (Comparative Examples 9 and 10)

The amounts of IFN-γ induced were determined according to similar manners to Examples 6 and 7, respectively, except that OK-432 was not added to the blood. The results are shown in Table 3.

**Table 3**

| | Carrier 1 | Concentration of OK-432 | Amount of IFN-γ induced |
|---|---|---|---|
| | Bulk volume (µL) | (KE/mL) | (pg/mL) |
| Example 6 | 50 | 0.1 | 251 |
| Example 7 | 500 | 0.1 | 435 |
| Comparative Example 8 | - | 0.1 | 72 |
| Comparative Example 9 | 50 | - | <40 |
| Comparative Example 10 | 500 | - | <40 |

### (Examples 8 to 15)

The amounts of IFN-γ induced were determined according to a similar manner to Example 2, except that carrier 1 (particulate polystyrene-based polymer material, manufactured by Mitsubishi Chemical Corp., trade name: DIAION HP-50), carrier 2 (particulate polystyrene-based polymer material, manufactured by Organo Corporation, part number: Amberlite XAD-2000), carrier 3 (particulate polystyrene-based polymer material, manufactured by Organo Corporation, part number: Amberlite XAD-2), carrier 4 (particulate polystyrene-based polymer material, manufactured by Organo Corporation, part number: Amberlite XAD-4), carrier 5 (particulate polystyrene-based polymer material, manufactured by Organo Corporation, part number: Amberlite XAD-16HP), carrier 6 (particulate polyacrylic ester-based polymer material, manufactured by Organo Corporation, part number: Amberlite XAD-7), carrier 7 (particulate polypropylene-based polymer material, pellets for injection molding), and carrier 8 (particulate polyvinyl chloride-based polymer material, pellets for injection molding) were respectively used. The results are shown in Table 4.

### (Comparative Example 11)

The amount of IFN-γ induced was determined according to a similar manner to Example 8, except that a carrier was not used and the amount of the OK-432-containing blood added to the tube was 1.5 mL. The results are shown in Table 4.

### (Comparative Examples 12 to 19)

The amounts of IFN-γ induced were determined according to similar manners to Examples 8 to 15, respectively, except that OK-432 was not added to the blood. The results are shown in Table 4.

**Table 4**

| | Carrier | Material | Concentration of OK-432 (KE/mL) | Amount of IFN-γ induced |
|---|---|---|---|---|
| | Bulk volume (50 µL) | | | (pg/mL) |
| Example 8 | Carrier 1 | Polystyrene | 0.1 | 360 |
| Example 9 | Carrier 2 | Polystyrene | 0.1 | 255 |
| Example 10 | Carrier 3 | Polystyrene | 0.1 | 238 |
| Example 11 | Carrier 4 | Polystyrene | 0.1 | 378 |
| Example 12 | Carrier 5 | Polystyrene | 0.1 | 205 |
| Example 13 | Carrier 6 | Polyacrylic ester | 0.1 | 303 |
| Example 14 | Carrier 7 | Polypropylene | 0.1 | 342 |
| Example 15 | Carrier 8 | Polyvinyl chloride | 0.1 | 356 |
| Comparative Example 11 | None | - | 0.1 | 104 |
| Comparative Example 12 | Carrier 1 | Polystyrene | - | <10 |
| Comparative Example 13 | Carrier 2 | Polystyrene | - | <10 |
| Comparative Example 14 | Carrier 3 | Polystyrene | - | <10 |
| Comparative Example 15 | Carrier 4 | Polystyrene | - | <10 |
| Comparative Example 16 | Carrier 5 | Polystyrene | - | <10 |
| Comparative Example 17 | Carrier 6 | Polyacrylic ester | - | <10 |
| Comparative Example 18 | Carrier 7 | Polypropylene | - | <10 |
| Comparative Example 19 | Carrier 8 | Polyvinyl chloride | - | <10 |

### (Examples 16 to 23)

The amounts of IFN-γ induced were determined according to a similar manner to Example 2, except that carrier 9 (petroleum pitch-based spherical active carbon, specific surface area: not less than 800 m²/g, particle diameter: 200 to 400 µm, manufactured by Kureha Chemical Industry Co., Ltd, trade name: Kremezin fine granule (for chronic renal failure) ) , carrier 10 (petroleum pitch-based spherical active carbon, specific surface area: 1100 to 1300 m²/g, particle diameter: 400 to 800 µm, manufactured by Kureha Chemical Industry Co., Ltd, trade name: BAC-LP), carrier 11 (phenol resin-based spherical active carbon, specific surface area: not less than 1000 m²/g, particle diameter: 150 to 300 µm, manufactured by Kanebo, Ltd., trade name: Bellfine AB), carrier 12 (petroleum pitch-based spherical active carbon, specific surface area: 800 to 1200 m²/g, particle diameter: 500 to 800 µm, manufactured by GL Sciences, trade name: Active Carbon Beads), carrier 13 (petroleum-based crushed active carbon, specific surface area: not less than 1000 m²/g, particle diameter: 400 to 1700 µm, manufactured by Meiwa Industries, Co. Ltd. , trade name: CG1040) , carrier 14 (coconut husk-based crushed active carbon, specific surface area: not less than 800 m²/g, particle diameter: 500 to 1800 µm, manufactured by Nacalai Tesque, Inc., trade name: Coconut husk active carbon (not washed) of 8 to 32 mesh) , carrier 15 (phenol resin-based columnar molecular sieve carbon, specific surface area: not less than 500 m²/g, particle diameter: 1000 to 4000 µm, manufactured by Kanebo, Ltd., trade name: Bellfine MG) , and carrier 16 (phenol resin-based columnar molecular sieve carbon, specific surface area: not less than 1500 m²/g, particle diameter: 1000 to 4000 µm, manufactured by Kanebo, Ltd., trade name: Bellfine BG) were respectively used. The results are shown in Table 5. The particle diameter refers to a minor axis diameter and a longer axis diameter.

### (Example 24 to 31)

The amounts of IFN-γ induced were determined according to a similar manner to Examples 16 to 23, except that carriers 9 to 16 were respectively crushed prior to use, using an agate mortar so that the particle diameter became not more than 100 µm. The results are shown in Table 5.

### (Comparative Example 20)

The amount of IFN-γ induced was determined according to a similar manner to Example 16, except that carrier was not used and the amount of the OK-432-containing blood added to the tube was 1.5 mL. The results are shown in Table 5.

### (Comparative Examples 21 to 28)

The amounts of IFN-γ induced were determined according to similar manners to Examples 16 to 23, respectively, except that OK-432 was not added to the blood. The results are shown in Table 5.

**Table 5**

| | Carrier Bulk volume (50 µL) | Trade name | Concentration of OK-432 (KE/mL) | Amount of IFN-γ induced (pg/mL) |
|---|---|---|---|---|
| Example 16 | Carrier 9 | Kremezin fine granule | 0. 1 | 526 |
| Example 17 | Carrier 10 | BAC-LP | 0. 1 | 495 |
| Example 18 | Carrier 11 | Bellfine AB | 0. 1 | 531 |
| Example 19 | Carrier 12 | Active Carbon Beads | 0. 1 | 488 |
| Example 20 | Carrier 13 | CG1040 | 0. 1 | 376 |
| Example 21 | Carrier 14 | Coconut husk Active Carbon | 0. 1 | 368 |
| Example 22 | Carrier 15 | Bellfine MG | 0. 1 | 305 |
| Example 23 | Carrier 16 | Bellfine BG | 0. 1 | 581 |
| Example 24 | Carrier 9 | Kremezin fine granule | 0. 1 | 230 |
| Example 25 | Carrier 10 | BAC-LP | 0. 1 | 206 |
| Example 26 | Carrier 11 | Bellfine AB | 0. 1 | 244 |
| Example 27 | Carrier 12 | Active Carbon Beads | 0.1 | 222 |
| Example 28 | Carrier 13 | CG1040 | 0. 1 | 217 |
| Example 29 | Carrier 14 | Coconut husk Active Carbon | 0. 1 | 231 |
| Example 30 | Carrier 15 | Bellfine MG | 0. 1 | 185 |
| Example 31 | Carrier 16 | Bellfine BG | 0. 1 | 255 |
| Comparative Example 20 | - | - | 0. 1 | 104 |
| Comparative Example 21 | Carrier 9 | Kremezin fine granule | - | <10 |
| Comparative Example 22 | Carrier 10 | BAC-LP | - | <10 |
| Comparative Example 23 | Carrier 11 | Bellfine AB | - | <10 |
| Comparative Example 24 | Carrier 12 | Active Carbon Beads | - - | <10 |
| Comparative Example 25 | Carrier 13 | CG1040 | - | <10 |
| Comparative Example 26 | Carrier 14 | Coconut husk Active Carbon | - | <10 |
| Comparative Example 27 | Carrier 15 | Bellfine MG | - | <10 |
| Comparative Example 28 | Carrier 16 | Bellfine BG | - | <10 |

### (Example 32)

Carrier 4 of polystyrene-divinylbenzene copolymer (particulate polystyrene-based polymer material, manufactured by Organo Corporation, part number: Amberlite XAD-4) in the particle bulk volume of 1 mL, OK-432 (manufactured by Chugai Pharmaceutical Co., Ltd., Trade Name: Picibanil, 1 KE/mL) and 1 mL of physiological saline comprising 0.1 vol% of formalin (neutral buffered formalin solution, manufactured by Wako Pure Chemical Industries, Ltd.) were mixed and tumbled to stir at 37°C for 20 hours, whereby OK-432 was physically adsorbed on the surface of the particles of the carrier 4. The particles were thereafter thoroughly washed with physiological saline and the particles in bulk volume of 100 µL were then packed in a sterilized tube (manufactured by DIATRON Corp., for 1.5 ml use) .

The amount of IFN-γ induced was determined according to a similar manner to Example 1, except that OK-432 was not added to the blood and the amount of the blood added to the tube was 1.4 mL. The results are shown in Table 6.

### (Examples 33 to 38)

The amounts of IFN-γ induced were determined according to a similar manner to Example 32, except that the concentrations of formalin (volume%) were as shown in Table 6. The results are shown in Table 6.

### (Comparative Example 29)

The amount of IFN-γ induced was determined according to a similar manner to Example 32, except that carrier 4 which had not been subjected to physical adsorption treatment with OK-432 was used. The results are shown in Table 6.

### (Comparative Example 30)

The amount of IFN-γ induced was determined according to a similar manner to Example 32, except that carrier 4 was not used and 1.5 mL of the OK-432 (0.1 KE/mL) -containing blood was added to the tube. The results are shown in Table 6.

**Table 6**

| | Carrier 4 Bulk volume (100 µL) | Concentration of OK-432 (KE/mL) | Amount of IFN-γ induced (pg/mL) |
|---|---|---|---|
| Example 32 | Formalin0. 1 % | - | 233 |
| Example 33 | Formalin0. 5% | - | 261 |
| Example 34 | Formalin1. 0% | - | 280 |
| Example 35 | Formalin2. 0% | - | 288 |
| Example 36 | Formalin3. 0% | - | 212 |
| Example 37 | Formalin5. 0% | - | 255 |
| Example 38 | Formalin10. 0% | - | 222 |
| Comparative Example 29 | No physical adsorption | - | <10 |
| Comparative Example 30 | - | 0.1 | 96 |

### (Example 39)

The carrier 4 in which OK-432 had been physically adsorbed on the surface of particles, which was prepared according to a similar manner to Example 34, in a bulk volume of 0.8 mL was packed in a blood bag (for 10 mL use). Blood was collected from a healthy human to obtain venous blood containing 15 IU/mL of heparin, and 10 mL of the venous blood was introduced into the blood bag. The blood bag was incubated while gently stirred at 37°C for 24 hours. The amount of IFN-γ induced in the blood was determined in the same manner as in Example 1. The results are shown in Table 7.

### (Examples 40 and 41)

The amounts of IFN-γ induced were determined according to a similar manner to Example 39, except that carrier 9 and carrier 16 were respectively used as a carrier and physiological saline free from formalin was used. The results are shown in Table 7.

### (Comparative Examples 31 to 33)

The amounts of IFN-γ induced were determined according to a similar manner to Examples 39 to 41, respectively, except that carriers 4, 9 and 16 which had not been subjected to physical adsorption treatment with OK-432 were respectively used. The results are shown in Table 7.

### (Comparative Example 34)

The amount of IFN-γ induced was determined according to a similar manner to Example 39, except that carrier 8 was not used and the amount of the OK-432 (0.1 KE/mL) -containing blood introduced in the blood bag was 10 mL. The results are shown in Table 7.

**Table 7**

| | Carrier 4 Bulk volume (0.8 mL) | Carrier 9 Bulk volume (0.8 mL) | Carrier 16 Bulk volume (0.8 mL) | Concentration of OK-432 (KE/mL) | Amount of IFN-y induced (pg/mL) |
|---|---|---|---|---|---|
| Example 39 | Physical adsorption | - | - | - | 257 |
| Example 40 | - | Physical adsorption | - | - | 377 |
| Example 41 | - | - | Physical adsorption | - | 420 |
| Comparative Example 31 | No physical adsorption | - | - | - | <10 |
| Comparative Example 32 | - | No physical adsorption | - | - | <10 |
| Comparative Example 33 | - | - | No physical adsorption | - | <10 |
| Comparative Example 34 | - | - | - | 0.1 | 98 |

### (Example 42)

The carrier 4 in which OK-432 had been physically adsorbed on the surface of particles according to a similar manner to Example 34 in a bulk volume of 4 mL was packed in a blood bag (for 50 mL use). Blood was collected from a healthy human to obtain venous blood containing 15 IU/mL of heparin, and 50 mL of the venous blood was introduced into the blood bag. The blood bag was incubated while gently stirred at 37°C for 24 hours. The amount of IFN-γ induced in the blood was determined in the same manner as in Example 1. The results are shown in Table 8.

### (Comparative Example 35)

The amount of IFN-γ induced was determined according to a similar manner to Example 42, except that carrier 4 which had not been subjected to physical adsorption treatment with OK-432 was used. The results are shown in Table 8.

### (Comparative Example 36)

The amount of IFN-γ induced in the plasma was determined according to a similar manner to Example 42, except that carrier 4 was not used and 50 mL of the OK-432 (0.1 KE/mL)-containing blood was introduced in the blood bag. The results are shown in Table 8.

**Table 8**

| | Carrier 4 Bulk volume (4 mL) | Concentration of OK-432 (KE/mL) | Amount of IFN-γ induced (pg/mL) |
|---|---|---|---|
| Example 42 | Physical adsorption | - | 316 |
| Comparative Example 35 | No physical adsorption | - | <10 |
| Comparative Example 36 | - | 0.1 | 102 |

### (Examples 43 and 44)

The amounts of IFN-γ induced were determined according to a similar manner to Example 2, except that the concentrations of OK-432 were two doses of 0.01 KE/mL and 0.1 KE/mL, respectively. The results are shown in Table 9.

### (Examples 45 and 46)

The amounts of IFN-γ induced were determined according to a similar manner to Example 2, except that carrier 9 was used as a carrier and the concentrations of OK-432 were two doses of 0.01 KE/mL and 0.1 KE/mL, respectively. The results are shown in Table 9.

### (Comparative Examples 37 and 38)

The amounts of IFN-γ induced were determined according to similar manners to Examples 43 and 45, respectively, except that OK-432 was not added to the blood. The results are shown in Table 9.

### (Comparative Examples 39 and 40)

The amounts of IFN-γ induced were determined according to similar manners to Examples 43 and 44, respectively, except that carrier 1 was not added and the amount of the OK-432-containing blood added to the tube was 1.5 mL. The results are shown in Table 9.

**Table 9**

| | Carrier 1 Bulk volume (µL) | Carrier 9 Bulk volume (µL) | Concentration of OK-432 (KE/mL) | Amount of IFN-γ induced (pg/mL) | | |
|---|---|---|---|---|---|---|
| | | | | Blood donor | | |
| | | | | A | B | C |
| Example 43 | 50 | - | 0. 01 | 79 | 355 | 128 |
| Example 44 | 50 | - | 0. 1 | 267 | 466 | 432 |
| Example 45 | - | 50 | 0. 01 | 145 | 394 | 249 |
| Example 46 | - | 50 | 0. 1 | 385 | 665 | 512 |
| Comparative Example 37 | 50 | - | - | <10 | 12 | <10 |
| Comparative Example 38 | - | 50 | - | <10 | <10 | <10 |
| Comparative Example 39 | - | - | 0. 01 | <10 | 94 | 59 |
| Comparative Example 40 | - | - | 0. 1 | 84 | 176 | 89 |

### (Example 47)

Carrier 16 (phenol resin-based columnar granulated active carbon, specific surface area: not less than 1500 m²/g, particle diameter: 1000 to 4000 µm, manufactured by Kanebo, Ltd., trade name: Bellfine BG) in a bulk volume of 25 mL was put in a 50 mL tube. A solution (25 mL) of 2% (v/v) of polyhydroxyethyl methacrylate (average molecular weight: 300,000, manufactured by SIGMA Corporation) in 95% ethanol (manufactured by Nacalai Tesque) was put in the tube and the carrier was soaked therein for 30 minutes. The carrier was transferred to a stainless basket to remove excess ethanol and then dried at 80°C for 20 hours to prepare carrier 17 on which poly(2-hydroxyethyl methacrylate) had been coated on the surface. The carrier 17 in a bulk volume of 1 mL was thereafter mixed with 1 mL of a suspension of OK-432 (manufactured by Chugai Pharmaceutical Co. , Ltd., Trade Name: Picibanil, 5 KE/mL) in physiological saline, and rotated to stir at 37°C for 20 hours, whereby OK-432 was physically adsorbed on the surface of particles of carrier 17. The particles were thereafter thoroughly washed with physiological saline and the particles in a bulk volume of 100 µL were then packed in a sterilized tube (manufactured by DIATRON Corp., for 1.5 ml use).

The amount of IFN-γ induced was determined according to a similar manner to Example 1, except that OK-432 was not added to the blood and the amount of the blood added to the tube was 1.4 mL. The results are shown in Table 10.

### (Comparative Example 41)

The amount of IFN-γ induced was determined according to a similar manner to Example 47, except that carrier 17 which had not been subjected to physical adsorption treatment with OK-432 was used. The results are shown in Table 10.

### (Comparative Examples 42 and 43)

The amounts of IFN-γ induced were determined according to a similar manner to Example 47, except that carrier 17 on which OK-432 had been physically adsorbed was not used and the amount of the OK-432-containing blood added to the tube was 1.5 mL, where the concentrations of OK-432 were two doses of 0.01 KE/mL and 0.1 KE/mL, respectively. The results are shown in Table 10.

**Table 10**

| | Carrier 17 Coated | Concentration of OK-432 (KE/mL) | Amount of IFN-γ induced (pg/mL) |
|---|---|---|---|
| Example 47 | Physically adsorbed | - | 551 |
| Comparative Example 41 | Not physically adsorbed | - - | 18 |
| Comparative Example 42 | - | 0.01 | 72 |
| Comparative Example 43 | - | 0.1 | 95 |

### INDUSTRIAL APPLICABILITY

The instrument for inducing a cytokine of the present invention comprises hemolytic streptococcus and/or a hemolytic streptococcus-origin component and a water-insoluble carrier. Therefore, where blood, a blood constituent or the like is added to the instrument, a cytokine can be induced quite effectively according to the method for inducing a cytokine of the present invention. Therefore, the present invention is very useful for the treatment of various diseases to which induction of a cytokine is effective.

## Claims

1. An instrument for inducing a cytokine, which comprises hemolytic streptococcus and/or a hemolytic streptococcus-origin component and a water-insoluble carrier.

2. The instrument for inducing a cytokine according to claim 1, which further comprises a container which comprises the hemolytic streptococcus and/or the hemolytic streptococcus-origin component and the water-insoluble carrier.

3. The instrument for inducing a cytokine according to claim 1 or 2, wherein the water-insoluble carrier has an effect for enhancing induction of a cytokine.

4. The instrument for inducing a cytokine according to any one of claims 1 to 3, wherein the hemolytic streptococcus and/or the hemolytic streptococcus-origin component have been fixed on the water-insoluble carrier.

5. The instrument for inducing a cytokine according to any one of claims 1 to 4, wherein the water-insoluble carrier comprises a polymer material.

6. The instrument for inducing a cytokine according to claim 5, wherein the polymer material is a porous polymer material.

7. The instrument for inducing a cytokine according to claim 5 or 6, wherein the polymer material comprises at least one kind selected from the group consisting of a polystyrene-based polymer material, a polyacrylic ester-based polymer material, a polypropylene-based polymer material and a polyvinyl chloride-based polymer material.

8. The instrument for inducing a cytokine according to any one of claims 1 to 4, wherein the water-insoluble carrier comprises a carbon material.

9. The instrument for inducing a cytokine according to claim 8, wherein the carbon material is an active carbon.

10. The instrument for inducing a cytokine according to claim 9, wherein the active carbon has a diameter of more than 100 µm and not more than 10000 µm.

11. The instrument for inducing a cytokine according to claim 9 or 10, wherein the active carbon is an active carbon obtained from at least one kind of raw material selected from the group consisting of a petroleum pitch, a phenolic resin, a coal and a coconut husk.

12. The instrument for inducing a cytokine according to any one of claims 1 to 11, which is used for the induction of a cytokine production in a cell which can produce a cytokine.

13. The instrument for inducing a cytokine according to claim 12, wherein the cell which can produce a cytokine is a cell derived from blood or a blood constituent.

14. A method for inducing a cytokine, which comprises inducing a cytokine using the instrument for inducing a cytokine according to any one of claims 1 to 13.
